# EUROPÄISCHE PATENTSCHRIFT

(11) **EP 3 747 346 B1**
(45) Veröffentlichungstag und Bekanntmachung des Hinweises auf die Patenterteilung: **27.12.2023**
(21) Anmeldenummer: 20172698.1
(22) Anmeldetag: 04.05.2020
(51) Int. Cl.: A61B 1/00, A61B 1/002, G02B 23/24, G02B 27/00

(54) **UMKEHRSATZ FÜR ENDOSKOP UND ENDOSKOP**
ENDOSCOPE AND REVERSE ASSEMBLY FOR ENDOSCOPE
ENSEMBLE D'INVERSION POUR UN ENDOSCOPE ET ENDOSCOPE

(30) Priorität: 06.06.2019 DE 102019115302
(43) Veröffentlichungstag der Anmeldung: 09.12.2020
(73) Patentinhaber: OLYMPUS WINTER & IBE GMBH, 22045 Hamburg (DE)
(72) Erfinder: ZHAO, Jianxin, 22399 Hamburg (DE)
(74) Vertreter: Seemann & Partner Patentanwälte mbB

(56) Entgegenhaltungen:
- WO-A1-95/35522
- DE-A1-102012 105 727
- DE-A1-102012 200 146
- DE-A1-102013 101 650
- US-A- 5 892 625

## Beschreibung

Die Erfindung betrifft einen Umkehrsatz für ein Endoskop, insbesondere Laparoskop oder Uroskop, sowie ein Endoskop, insbesondere Laparoskop oder Uroskop.

Im Stand der Technik ist bekannt, dass starre Endoskope üblicherweise ein optisches System aufweisen, das aus einem Objektiv, einem Okular und aus einem zwischen diesen angeordneten Relaislinsensystem besteht, wobei das Relaislinsensystem mehrere Umkehrsätze aufweist. Üblicherweise ist eine ungerade Anzahl von Umkehrsätzen vorgesehen, da das Objektiv und jeder Umkehrsatz ein umgekehrtes Bild erzeugt und ein normales Endoskop ein aufrechtes Bild erzeugen soll, wodurch das erzeugte Bild aufrecht ausgerichtet ist. Um wesentliche Abbildungsfehler zu korrigieren, ist ein symmetrischer Aufbau von Umkehrsätzen vorgesehen.

Beispielsweise ist aus DE 11 2004 002 220 B4 ein Umkehrsatz für ein optisches System eines starren Endoskops bekannt. Ferner beschreibt DE 10 2012 200 146 A1 einen Umkehrsatz für ein Endoskop mit mehreren gleichartigen Umkehrsätzen.

DE 10 2013 101 650 A1 beschreibt ferner ein Optikrohr für ein Endoskop, wobei im Inneren des Optikrohrs mindestens eine Stablinse aufgenommen ist und auf der Stablinse eine Hülse fixiert ist. Ferner ist die Hülse mit dem Objektivrohr verschweißt.

Außerdem offenbart DE 10 2012 105 727 A1 eine Stablinseneinheit für ein Stablinsensystem für ein Endoskop, wobei in einer Hülse eine Stablinse angeordnet ist und mit der Hülse gefügt ist. Ferner ist ein Rand der Hülse nicht bündig mit einem Ende der Stablinse.

Ferner ist in WO 95/35522 A1 eine Relaislinsenanordnung mit mehreren Linsenbaugruppen beschrieben, wobei jede Linsenbaugruppe jeweils feste Linsen mit einer jeweils dazwischen angeordneten flüssigen Linse aufweist (vgl. Fig. 2).

Darüber hinaus ist in US 5,892,625 A eine Anordnung von Linsen für ein Endoskop gezeigt (vgl. Fig. 12). Hierbei sind an beiden Enden einer Hülse Achromate, die aus bikonvexen Linsen bzw. aus plankonvexen Linsen gebildet sind, angeordnet. Zwischen den Achromaten ist ein optisches Fluid in die Hülse eingebracht.

Ferner ist bekannt, dass in Laparoskopen Umkehrsätze eingesetzt werden. Bevorzugt werden hierbei sogenannte kurze Umkehrsätze verwendet. Die kurzen Umkehrsätze enthalten hierbei zwei symmetrisch und aus zwei Achromaten bestehende Linsengruppen, die zusammen in einem Systemrohr montiert sind, wobei zwischen den Achromaten der beiden Linsengruppen Abstandshalter angeordnet sind. Es hat sich gezeigt, dass die kurzen Umkehrsätze in den Laparoskopen insbesondere instabil im Aufbau sind.

Eine Aufgabe der Erfindung besteht darin, Umkehrsätze für Endoskope sowie ein Endoskop mit entsprechenden Umkehrsätzen bereitzustellen, bei denen auf einfache Weise die optische Performanz verbessert wird bzw. ist.

Gelöst wird diese Aufgabe durch einen Umkehrsatz für ein Endoskop, insbesondere Laparoskop oder Uroskop, mit zwei jeweils wenigstens zwei Linsen aufweisenden äußeren Achromaten, wobei die äußeren Achromate einen Außendurchmesser aufweisen, und mit zwei zwischen den äußeren Achromaten angeordneten inneren Achromaten, wobei jeder der inneren Achromate wenigstens zwei Linsen aufweist, wobei die Linsen der inneren Achromate in einer Aufnahmehülse aufgenommen sind, wobei die Aufnahmehülse mit den beiden inneren Achromaten zwischen den äußeren Achromaten angeordnet ist.

Die Erfindung beruht auf dem Gedanken, dass durch die Anordnung der beiden inneren Achromate in der Aufnahmehülse eine Verkippung der inneren Achromate, die zwischen den beiden äußeren Achromaten angeordnet sind, reduziert oder vermieden wird. Dadurch, dass die beiden inneren Achromate sicher in der Aufnahmehülse positioniert und ausgerichtet sind, werden die inneren Achromate auf einfache Weise beispielsweise unter Verwendung einer Zentriervorrichtung in der als Fassung für die inneren Achromate ausgebildeten Aufnahmehülse angeordnet.

Dazu ist vorgesehen, dass die Außendurchmesser der inneren Achromate kleiner sind als die Außendurchmesser eines oder der beiden äußeren Achromate. Insbesondere sind die äußeren Achromate angrenzend an die oder gegenüberliegend von den Enden der Aufnahmehülse angeordnet. Darüber hinaus ist vorgesehen, dass die äußeren Achromate und die Aufnahmehülse mit den beiden inneren Achromaten von einem Systemrohr eines Endoskops, wie zum Beispiel einem Faserrohr, oder von einer, vorzugsweise gemeinsamen Halterung umgeben sind bzw. darin nebeneinander bzw. bezogen auf die optische Achse des Endoskops hintereinander angeordnet sind.

Im Rahmen der Erfindung ist vorgesehen, dass die äußeren Achromate sowie die inneren Achromate als, insbesondere verklebte oder verkittete, Duplett-Achromate oder Triplett-Achromate ausgebildet sind.

Insgesamt wird durch den erfindungsgemäßen Umkehrsatz eine Endoskopoptik eines Endoskops verbessert, da die optische Performanz erhöht wird.

Gemäß einer Weiterbildung des Umkehrsatzes ist es bevorzugt, dass die inneren Achromate jeweils einen Außendurchmesser aufweisen, wobei der Außendurchmesser eines inneren Achromats oder der Außendurchmesser der inneren Achromate kleiner ist als die Außendurchmesser der, insbesondere dem jeweiligen inneren Achromat gegenüberliegenden, äußeren Achromaten.

Gemäß einem weiteren Aspekt zeichnet sich der Umkehrsatz dadurch aus, dass die Aufnahmehülse einen Außendurchmesser aufweist, der dem Außendurchmesser eines äußeren Achromats oder dem Außendurchmesser der beiden äußeren Achromate entspricht. Hierdurch wird ermöglicht, dass die äußeren Achromate und die zwischen den äußeren Achromaten angeordnete Aufnahmehülse, in dem die beiden inneren Achromate aufgenommen sind, in einem Systemrohr zum Beispiel eines Endoskops hintereinander angeordnet werden oder sind.

Vorzugsweise sind die Linsen eines inneren Achromaten oder die Linsen der beiden inneren Achromate in der Aufnahmehülse eingeklebt bzw. verkittet.

Darüber hinaus ist gemäß einer Weiterbildung des Umkehrsatzes vorgesehen, dass bei Aufnahme oder Anordnung von zwei inneren bzw. der beiden inneren Achromaten in der Aufnahmehülse die Aufnahmehülse im Inneren zwischen den inneren Achromaten eine Anschlagbarriere für die beiden inneren Achromate aufweist. Durch die Anschlagbarriere im Innenraum der Aufnahmehülse wird ein Anschlag für die jeweils von einer Seite eingeführten inneren Achromate bereitgestellt, wobei außerdem durch die Anschlagbarriere der Abstand zwischen den beiden in der Aufnahmehülse angeordneten inneren Achromate konstant ist.

Außerdem ist es bei einer Ausgestaltung des Umkehrsatzes vorteilhaft, dass ein äußerer Achromat oder die äußeren Achromate jeweils eine Zerstreuungslinse mit einer Dicke aufweisen, wobei die Dicke der Zerstreuungslinse des äußeren Achromats oder die Dicken der Zerstreuungslinsen der äußeren Achromate um den Faktor von wenigstens 1,5, insbesondere um den Faktor von mehr als 2, größer ist als der Außendurchmesser des äußeren Achromats oder der äußeren Achromate. Die Zerstreuungslinsen sind vorzugsweise als bikonkave Linsen mit einer negativen Brennweite ausgebildet. Insbesondere sind die Zerstreuungslinsen der äußeren Achromate den Enden der Aufnahmehülse gegenüberliegend, zum Beispiel in einem Systemrohr, angeordnet.

Außerdem ist gemäß einer bevorzugten Weiterbildung vorgesehen, dass ein Ende der Aufnahmehülse, das einem äußeren Achromaten zugewandt ist, oder die beiden Enden der Aufnahmehülse, die jeweils einem äußeren Achromat zugewandt sind, eine plane Anlagefläche für die jeweils dem Ende der Aufnahmehülse zugewandte Linse des äußeren Achromats oder jeweils eine Anlagefläche für die jeweils den Enden der Aufnahmehülse zugewandten Linsen der jeweiligen äußeren Achromate aufweisen. Dadurch ist es beispielsweise möglich, dass die Linse des äußeren Achromats, die dem Ende der Aufnahmehülse gegenüberliegend angeordnet ist, mit dem Ende der Aufnahmehülse in Kontakt gebracht wird oder ist. Insgesamt ergibt sich dadurch eine exakte Ausrichtung und Positionierung der beiden äußeren Achromate zusammen mit der Aufnahmehülse. Außerdem wird dadurch eine Verkippung der äußeren Achromate zu der Aufnahmehülse vermieden.

Ferner zeichnet sich eine Ausgestaltung des Umkehrsatzes dadurch aus, dass die Linse, insbesondere Zerstreuungslinse, des äußeren Achromats, die der Aufnahmehülse zugewandt ist, eine flache, insbesondere umlaufende, Anlageschulter aufweist oder dass die Linsen, insbesondere Zerstreuungslinsen, der äußeren Achromate, die jeweils der Aufnahmehülse zugewandt sind, jeweils eine flache, insbesondere umlaufende, Anlageschulter aufweisen. Im Rahmen der Erfindung kann dabei vorgesehen sein, dass die Linsen der äußeren Achromate mit den Enden der Aufnahmehülse verbunden bzw. verklebt werden, wodurch eine hohe Stabilität des Umkehrsatzes erreicht wird.

Gemäß einem weiteren Gesichtspunkt ist vorgesehen, dass die Aufnahmehülse mit einem äußeren Achromat oder mit den beiden äu-ßeren Achromaten verklebt ist.

Ferner ist in einer Weiterbildung des Umkehrsatzes vorgesehen, dass ein Paar mit einem äußeren Achromat und einem inneren Achromat symmetrisch zu einem zweiten Paar mit einem äußeren Achromat und einem inneren Achromat ausgebildet ist.

Überdies ist es gemäß einem Aspekt bevorzugt, dass die äußeren Achromate und die inneren Achromate in einem Systemrohr, insbesondere Systemrohr des Endoskops, angeordnet sind.

Die Aufgabe wird ferner gelöst durch ein Endoskop, insbesondere Laparoskop oder Uroskop, mit wenigstens einem Umkehrsatz, wobei das Endoskop mit wenigstens einem voranstehend beschriebenen Umkehrsatz ausgebildet ist. Zur Vermeidung von Wiederholungen wird auf die obigen Ausführungen ausdrücklich verwiesen.

Insbesondere weist das Endoskop eine Endoskopoptik auf, die ein distales Objektiv sowie eine proximale Bildbetrachtungsvorrichtung, zum Beispiel ein Okular, aufweist. Darüber hinaus ist zwischen dem Objektiv und der Bildbetrachtung in einem Rohr, insbesondere Endoskopschaft, als Bildleiter ein Relaislinsensystem vorgesehen, das vorzugsweise mehrere hintereinander angeordnete Umkehrsätze aufweist.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand eines Ausführungsbeispiels unter Bezugnahme auf die Zeichnung beschrieben, wobei bezüglich aller im Text nicht näher erläuterten erfindungsgemäßen Einzelheiten ausdrücklich auf die Zeichnung verwiesen wird. Es zeigt:

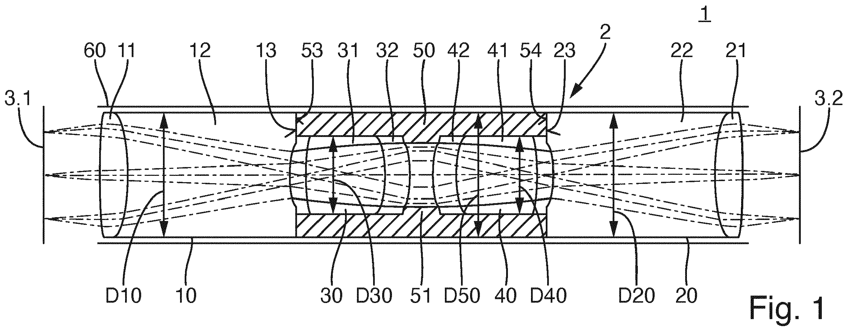

- Fig. 1: schematisch eine Darstellung eines erfindungsgemäßen Umkehrsatzes für ein Endoskop.

In Fig. 1 ist schematisch ein Umkehrsatz 2 für ein schematisch bezeichnetes Endoskop 1 dargestellt. Der Umkehrsatz 2 ist hierbei Bestandteil eines optischen Systems des Endoskops 1. Vorzugsweise ist das Endoskop 1 als starres Endoskop ausgebildet. Das optische System des Endoskops 1 ist hierbei in einem, vorzugsweise starren, Rohr (nicht dargestellt) angeordnet. Das optische Systems des Endoskops 1 umfasst hierbei am distalen Ende ein Objektiv, wobei das vom Objektiv erzeugte Bild beispielsweise unter Verwendung mehrerer Umkehrsätze zu einer Bildebene am proximalen Ende übertragen wird, auf der das Bild von einem Okular betrachtet wird. Am Okular kann auch eine Kamera angeordnet sein.

Vorzugsweise weist das optische System des Endoskops 1 mehrere, insbesondere eine ungerade Anzahl an Umkehrsätzen auf, wodurch ein aufrechtes Bild in der letzten proximalen Bildebene vor dem Okular erzeugt wird.

Der in Fig. 1 dargestellte Umkehrsatz 2 des optischen Systems des Endoskops 1 überträgt von einer ersten Bildebene 3.1 das Bild auf eine zweite Bildebene 3.2. Das Bild, das in der Bildebene 3.1 vorhanden ist, wird auf bzw. in der Bildebene 3.2 umgekehrt dargestellt. Dabei ist das Bild der Bildebene 3.1 in der Bildebene 3.2 invertiert.

Der Umkehrsatz 2 weist zwei äußere Achromate 10, 20 auf, zwischen denen zwei innere Achromate 30, 40 angeordnet sind. Der äußere Achromat 10 umfasst hierbei eine bikonvexe Linse 11 und eine bikonkave Linse 12. Entsprechend weist der äußere Achromat 20 eine bikonvexe Linse 21 und eine bikonkave Linse 22 auf. Die Linsen 11, 12 des äußeren Achromats 10 sowie die Linsen 21, 22 des zweiten äußeren Achromaten 20 sind hierbei im Querschnitt, d.h. senkrecht zur Zeichenebene kreisrund ausgebildet und weisen hierbei einen Außendurchmesser D10 bzw. D20 auf.

Die zwischen den äußeren Achromaten 10, 20 angeordneten inneren Achromate 30, 40 weisen jeweils eine bikonvexe Linse 31, 41 sowie eine konvexkonkave Linse 32 bzw. 42 auf.

Die Linsen 31, 32 des inneren Achromats 30 bzw. die Linsen 41, 42 des zweiten inneren Achromats 40 sind im Querschnitt kreisrund ausgebildet und weisen einen Durchmesser D30 sowie D40 auf. Insbesondere entspricht der Durchmesser D30 der Linsen 31, 32 dem Durchmesser D40 der Linsen 41, 42 des zweiten inneren Achromats 40.

Die inneren Achromate 30, 40 bzw. die Linsen 31, 32, 41, 42 der inneren Achromate 30, 40 sind in einer Hülse 50 angeordnet und darin aufgenommen. Vorzugsweise sind die Linsen 31, 32, 41, 42 der inneren Achromate 30, 40 in der Hülse 50 eingeklebt. Darüber hinaus ist in der Hülse 50 zwischen den inneren Achromaten 30, 40 eine radiale nach innen ragende Anschlagbarriere 51 für die Linsen 32, 42 ausgebildet. Dadurch sind die Linsen 32, 42 der Achromate 30, 40 voneinander beabstandet. Die Hülse 50 ist im Querschnitt kreisrund ausgebildet und weist einen Außendurchmesser D50 auf. Vorzugsweise entspricht der Außendurchmesser D50 der Hülse 50 den Außendurchmessern D10 und D20 der äußeren Achromate 10, 20 bzw. deren Linsen 11, 12 bzw. 21, 22.

Vorzugsweise sind die Linsen 31, 32 des Achromaten 30 sowie die Linsen 41, 42 des Achromaten 40 jeweils verkittet.

Die Hülse 50 weist an ihren zu den äußeren Achromaten 10, 20 weisenden Enden jeweils eine Anlagefläche 53, 54 auf. Die Linsen 12, 22 der äußeren Achromaten 10, 20 sind länglich bzw. stabförmig ausgebildet und weisen (bezogen auf die optische Achse) eine Länge bzw. Dicke auf, die vorzugsweise mindestens um den Faktor 1,5 größer ist als der jeweilige Außendurchmesser D10 bzw. D20 der Linse 12 bzw. 22, bzw. der äußeren Achromate 10, 20.

An den der Hülse 50 zugewandten Seiten sind die Linsen 12 bzw. 22 mit einer flachen Schulterfläche 13 bzw. 23 ausgebildet, so dass die Schulterflächen 13 bzw. 23 der Linsen 12, 22 den Anlageflächen 53, 54 der Hülse gegenüber angeordnet sind und mit diesen gegebenenfalls verbunden sind. Die Schulterflächen 13, 23 der Linsen 12, 22 sind hierbei in Kontakt mit den Anlageflächen 53, 54 der Hülse 50.

Insbesondere sind die äußeren Achromate 10, 20 sowie die inneren Achromate 30, 40 in einem schematisch dargestellten Systemrohr 60 des Endoskops 1 angeordnet, so dass die Achromate 10, 20, 30, 40 von dem Systemrohr 60, insbesondere Faserrohr, umgeben sind.

Alle genannten Merkmale, auch die der Zeichnung allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 1: Endoskop
- 2: Umkehrsatz
- 3.1, 3.2: Bildebene
- 10: Achromat
- 11: bikonvexe Linse
- 12: bikonkave Linse
- 13: Schulterfläche
- 20: Achromat
- 21: bikonvexe Linse
- 22: bikonkave Linse
- 23: Schulterfläche
- 30: innerer Achromat
- 31: bikonvexe Linse
- 32: konvex-konkave Linse
- 40: innerer Achromat
- 41: bikonvexe Linse
- 42: konvex-konkave Linse
- 50: Hülse
- 51: Anschlagbarriere
- 53: Anlagefläche
- 54: Anlagefläche
- 60: Systemrohr

- D10: Außendurchmesser
- D20: Außendurchmesser
- D30: Außendurchmesser
- D40: Außendurchmesser
- D50: Außendurchmesser

## Patentansprüche

1. Umkehrsatz (2) für ein Endoskop (1), insbesondere Laparoskop oder Uroskop, mit zwei jeweils wenigstens zwei Linsen (11, 12; 21; 22) aufweisenden äußeren Achromaten (10, 20), wobei die äußeren Achromate (10, 20) einen Außendurchmesser (D10, D20) aufweisen, und mit zwei zwischen den äußeren Achromaten (10, 20) angeordneten inneren Achromaten (30, 40), wobei jeder der inneren Achromate (30, 40) wenigstens zwei Linsen (31, 32; 41, 42) aufweist, wobei die Linsen (31, 32; 41, 42) der beiden inneren Achromate (30, 40) in einer Aufnahmehülse (50) aufgenommen sind, wobei die Aufnahmehülse (50) mit den beiden inneren Achromaten (30, 40) zwischen den äußeren Achromaten (10, 20) angeordnet ist.

2. Umkehrsatz (2) nach Anspruch 1, **dadurch gekennzeichnet, dass** die inneren Achromate (30, 40) jeweils einen Außendurchmesser (D30, D40) aufweisen, wobei der Außendurchmesser (D30, D40) eines inneren Achromats (30, 40) oder der Außendurchmesser (D30, D40) der inneren Achromate (30, 40) kleiner ist als die Außendurchmesser (D10, D20) der, insbesondere dem jeweiligen inneren Achromat (30, 40) gegenüberliegenden, äu-ßeren Achromaten (10, 20).

3. Umkehrsatz (2) nach Anspruch 1 oder 2, **dadurch gekennzeichnet, dass** die Aufnahmehülse (50) einen Außendurchmesser (D50) aufweist, der dem Außendurchmesser (D10, D20) eines äußeren Achromats (D10, D20) oder dem Außendurchmesser (D10, D20) der beiden äußeren Achromate (10, 20) entspricht.

4. Umkehrsatz (2) nach einem der Ansprüche 1 bis 3, **dadurch gekennzeichnet, dass** die Linsen (11, 12; 21, 22) eines inneren Achromaten (30, 40) oder die Linsen (11, 12; 21, 22) der beiden inneren Achromate (30, 40) in der Aufnahmehülse (50) eingeklebt sind.

5. Umkehrsatz (2) nach einem der Ansprüche 1 bis 4, **dadurch gekennzeichnet, dass** bei Aufnahme von zwei inneren Achromaten (30, 40) in der Aufnahmehülse (50) die Aufnahmehülse (50) im Inneren zwischen den inneren Achromaten (30, 40) eine Anschlagbarriere (51) für die beiden inneren Achromate (30, 40) aufweist.

6. Umkehrsatz (2) nach einem der Ansprüche 1 bis 5, **dadurch gekennzeichnet, dass** ein äußerer Achromat (10, 20) oder die äu-ßeren Achromate (10, 20) jeweils eine Zerstreuungslinse (12, 22) mit einer Dicke weisen, wobei die Dicke der Zerstreuungslinse (12, 22) des äußeren Achromats (10, 20) oder die Dicken der Zerstreuungslinsen (12, 22) der äußeren Achromate (10, 20) um den Faktor von wenigstens 1,5, insbesondere um den Faktor von mehr als 2, größer ist als der Außendurchmesser (D10, D20) des äußeren Achromats (10, 20) oder der äußeren Achromate (10, 20).

7. Umkehrsatz (2) nach einem der Ansprüche 1 bis 6, **dadurch gekennzeichnet, dass** ein Ende der Aufnahmehülse (50), das einem äußeren Achromaten (10, 20) zugewandt ist, oder die beiden Enden der Aufnahmehülse (50), die jeweils einem äußeren Achromat (10, 20) zugewandt sind, eine plane Anlagefläche (53, 54) für die jeweils dem Ende der Aufnahmehülse (50) zugewandte Linse (12, 22) des äußeren Achromats (10, 20) oder jeweils eine Anlagefläche (53, 54) für die jeweils den Enden der Aufnahmehülse (50) zugewandten Linsen (12, 22) der jeweiligen äußeren Achromate (10, 20) aufweisen.

8. Umkehrsatz (2) nach einem der Ansprüche 1 bis 7, **dadurch gekennzeichnet, dass** die Linse (12, 22) des äußeren Achromats (10, 20), die der Aufnahmehülse (50) zugewandt ist, eine flache, insbesondere umlaufende, Anlageschulter (13, 23) aufweist oder dass die Linsen (12, 22) der äußeren Achromate (10, 20), die jeweils der Aufnahmehülse (50) zugewandt sind, jeweils eine flache, insbesondere umlaufende, Anlageschulter (13, 23) aufweisen.

9. Umkehrsatz (2) nach einem der Ansprüche 1 bis 8, **dadurch gekennzeichnet, dass** die Aufnahmehülse (50) mit einem äußeren Achromat (10, 20) oder mit den beiden äußeren Achromaten (10, 20) verklebt ist.

10. Umkehrsatz (2) nach einem der Ansprüche 1 bis 9, **dadurch gekennzeichnet, dass** ein Paar mit einem äußeren Achromat (10, 20) und einem inneren Achromat (30, 40) symmetrisch zu einem zweiten Paar mit einem äußeren Achromat (10, 20) und einem inneren Achromat (30, 40) ausgebildet ist.

11. Umkehrsatz (2) nach einem der Ansprüche 1 bis 10, **dadurch gekennzeichnet, dass** die äußeren Achromate (10, 20) und die inneren Achromate (30, 40) in einem Systemrohr (60), insbesondere Systemrohr (60) des Endoskops (1), angeordnet sind.

12. Endoskop (1), insbesondere Laparoskop oder Uroskop, mit wenigstens einem Umkehrsatz (2), wobei das Endoskop (1) mit wenigstens einem Umkehrsatz (2) nach einem der Ansprüche 1 bis 11 ausgebildet ist.

## Claims

1. A reversal system (2) for an endoscope (1), in particular a laparoscope or uroscope, with two outer achromats (10, 20) each having at least two lenses (11, 12; 21; 22), wherein the outer achromats (10, 20) have an outer diameter (D10, D20), and with two inner achromats (30, 40) arranged between the outer achromats (10, 20), wherein each of the inner achromats (30, 40) has at least two lenses (31, 32; 41, 42), wherein the lenses (31, 32; 41, 42) of the both inner achromats (30, 40) are accommodated in a holding sleeve (50), wherein the holding sleeve (50) with the both inner achromats (30, 40) is arranged between the outer achromats (10, 20).

2. The reversal system (2) according to claim 1, **characterized in that** the inner achromats (30, 40) each have an outer diameter (D30, D40), wherein the outer diameter (D30, D40) of an inner achromat (30, 40), or the outer diameter (D30, D40) of the inner achromats (30, 40), is smaller than the outer diameter (D10, D20) of the outer achromats (10, 20), in particular of the outer achromats (10, 20) opposite the respective inner achromat (30, 40).

3. The reversal system (2) according to claim 1 and 2, **characterized in that** the holding sleeve (50) has an outer diameter (D50) which corresponds to the outer diameter (D10, D20) of an outer achromat (D10, D20), or to the outer diameter (D10, D20) of the two outer achromats (10, 20).

4. The reversal system (2) according to one of claims 1 to 3, **characterized in that** the lenses (11, 12; 21, 22) of one inner achromat (30, 40) or the lenses (11, 12; 21, 22) of the two inner achromats (30, 40), are glued in the holding sleeve (50).

5. The reversal system (2) according to one of claims 1 to 4, **characterized in that** when two inner achromats (30, 40) are accommodated in the holding sleeve (50), the holding sleeve (50) has a stop barrier (51) for the two inner achromats (30, 40) in the interior between the inner achromats (30, 40).

6. The reversal system (2) according to one of claims 1 to 5, **characterized in that** an outer achromat (10, 20), or the outer achromats (10, 20), each have a concave lens (12, 22) with a thickness, wherein the thickness of the concave lens (12, 22) of the outer achromat (10, 20), or the thicknesses of the concave lenses (12, 22) of the outer achromats (10, 20), is/are greater by a factor of at least 1.5, in particular by a factor of more than 2, than the outer diameter (D10, D20) of the outer achromat (10, 20) or the outer achromats (10, 20).

7. The reversal system (2) according to one of claims 1 to 6, **characterized in that** one end of the holding sleeve (50) that faces an outer achromat (10, 20), or the two ends of the holding sleeve (50) that each face an outer achromat (10, 20), have a flat bearing surface (53, 54) for the lens (12, 22) of the outer achromat (10, 20) facing the end of the holding sleeve (50) in each case, or a bearing surface (53, 54) for the lenses (12, 22) of the respective outer achromats (10, 20) facing the ends of the holding sleeve (50) in each case.

8. The reversal system (2) according to one of claims 1 to 7, **characterized in that** the lens (12, 22) of the outer achromat (10, 20) which faces the holding sleeve (50) has a flat, in particular peripheral, bearing shoulder (13, 23), or that the lenses (12, 22) of the outer achromats (10, 20) that each face the holding sleeve (50) each have a flat, in particular peripheral, bearing shoulder (13, 23).

9. The reversal system (2) according to one of claims 1 to 8, **characterized in that** the holding sleeve (50) is glued to an outer achromat (10, 20), or to the two outer achromats (10, 20).

10. The reversal system (2) according to one of claims 1 to 9, **characterized in that** a pair having an outer achromat (10, 20) and an inner achromat (30, 40) is designed symmetrical to a second pair having an outer achromat (10, 20) and an inner achromat (30, 40).

11. The reversal system (2) according to one of claims 1 to 10, **characterized in that** the outer achromats (10, 20) and the inner achromats (30, 40) are arranged in a system tube (60), in particular a system tube (60) of the endoscope (1).

12. An endoscope (1), in particular a laparoscope or uroscope, having at least one reversal system (2), wherein the endoscope (1) is designed with at least one reversal system (2) according to one of claims 1 to 11.

## Revendications

1. Ensemble d'inversion (2) pour un endoscope (1), en particulier un laparoscope ou un uroscope, avec deux achromates extérieurs (10, 20) présentant chacun au moins deux lentilles (11, 12; 21; 22), les achromates extérieurs (10, 20) présentant un diamètre extérieur (D10, D20), et avec deux achromates intérieurs (30, 40) agencés entre les achromates extérieurs (10, 20), chacun des achromates intérieurs (30, 40) présentant au moins deux lentilles (31, 32; 41, 42), les lentilles (31, 32; 41, 42) des deux achromates intérieurs (30, 40) étant montées dans un manchon de réception (50), le manchon de réception (50) avec les deux achromates intérieurs (30, 40) étant agencé entre les achromates extérieurs (10, 20).

2. Ensemble d'inversion (2) selon la revendication 1, **caractérisé en ce que** les achromates intérieurs (30, 40) présentent chacun un diamètre extérieur (D30, D40), le diamètre extérieur (D30, D40) d'un achromate intérieur (30, 40) ou le diamètre extérieur (D30, D40) des achromates intérieurs (30, 40) étant plus petit que les diamètres extérieurs (D10, D20) des achromates extérieurs (10, 20), en particulier ceux qui sont opposés à l'achromate intérieur (30, 40) respectif.

3. Ensemble d'inversion (2) selon la revendication 1 ou la revendication 2, **caractérisé en ce que** le manchon de réception (50) présente un diamètre extérieur (D50) qui correspond au diamètre extérieur (D10, D20) d'un achromate extérieur (D10, D20) ou au diamètre extérieur (D10, D20) des deux achromates extérieurs (10, 20).

4. Ensemble d'inversion (2) selon l'une des revendications 1 à 3, **caractérisé en ce que** les lentilles (11, 12; 21, 22) d'un achromate interne (30, 40) ou les lentilles (11, 12; 21, 22) des deux achromates internes (30, 40) sont collées dans le manchon de réception (50).

5. Ensemble d'inversion (2) selon l'une des revendications 1 à 4, **caractérisé en ce que**, lorsque deux achromates internes (30, 40) sont reçus dans le manchon de réception (50), le manchon de réception (50) présente à l'intérieur de lui, entre les achromates internes (30, 40), une barrière de butée (51) pour les deux achromates internes (30, 40).

6. Ensemble d'inversion (2) selon l'une des revendications 1 à 5, **caractérisé en ce qu'**un achromate externe (10, 20) ou les achromates externes (10, 20) présentent chacun une lentille divergente (12, 22) d'une épaisseur telle que l'épaisseur de la lentille divergente (12, 22) de l'achromate externe (10, 20) ou les épaisseurs des lentilles divergentes (12, 22) des achromates extérieurs (10, 20) sont supérieures d'un facteur d'au moins 1,5, en particulier d'un facteur supérieur à 2, au diamètre extérieur (D10, D20) de l'achromate extérieur (10, 20) ou des achromates extérieurs (10, 20).

7. Ensemble d'inversion (2) selon l'une quelconque des revendications 1 à 6, **caractérisé en ce qu'**une extrémité du manchon de réception (50) est tournée vers un achromate externe (10, 20), ou les deux extrémités du manchon de réception (50) qui sont respectivement tournées vers un achromate externe (10, 20), présentent une surface d'appui plane (53, 54) pour la lentille (12, 22) respective de l'achromate extérieur (10, 20) tournée vers l'extrémité du manchon de réception (50), ou une surface d'appui (53, 54) respective pour les lentilles respectives (12, 22) des achromates extérieurs respectifs (10, 20) tournées vers les extrémités du manchon de réception (50).

8. Ensemble d'inversion (2) selon l'une des revendications 1 à 7, **caractérisé en ce que** la lentille (12, 22) de l'achromate extérieur (10, 20) qui est tournée vers le manchon de réception (50) présente un épaulement d'appui plat (13, 23), en particulier périphérique, ou **en ce que** les lentilles (12, 22) des achromates extérieurs (10, 20), qui sont respectivement tournées vers le manchon de réception (50), présentent chacune un épaulement d'appui plat (13, 23), en particulier périphérique.

9. Ensemble d'inversion (2) selon l'une des revendications 1 à 8, **caractérisé en ce que** le manchon de réception (50) est collé à un achromate extérieur (10, 20) ou aux deux achromates extérieurs (10, 20).

10. Ensemble d'inversion (2) selon l'une des revendications 1 à 9, **caractérisé en ce qu'**une paire comprenant un achromate extérieur (10, 20) et un achromate intérieur (30, 40) est formée symétriquement par rapport à une deuxième paire comprenant un achromate extérieur (10, 20) et un achromate intérieur (30, 40).

11. Ensemble d'inversion (2) selon l'une des revendications 1 à 10, **caractérisé en ce que** les achromates externes (10, 20) et les achromates internes (30, 40) sont agencés dans un tube système (60), en particulier un tube système (60) de l'endoscope (1).

12. Endoscope (1), en particulier laparoscope ou uroscope, avec au moins un ensemble d'inversion (2), l'endoscope (1) étant réalisé avec au moins un ensemble d'inversion (2) selon l'une des revendications 1 à 11.
